# EUROPEAN PATENT SPECIFICATION

(11) **EP 4 337 279 B1**
(45) Date of publication and mention of the grant of the patent: **24.06.2026**
(21) Application number: 22728229.0
(22) Date of filing: 10.05.2022
(51) Int. Cl.: A61M 1/06, A61M 1/00

(54) **BREAST PUMP**
MILCHPUMPE
TIRE-LAIT

(30) Priority: 10.05.2021 EP 21173018
(43) Date of publication of application: 20.03.2024
(73) Proprietor: Medela Holding AG, 6340 Baar (CH)
(72) Inventor: XU, Chen, Shanghai 200233 (CN)
(74) Representative: Grünecker Patent- und Rechtsanwälte PartG mbB
(86) International application number: PCT/EP2022/062527
(87) International publication number: WO 2022/238348

(56) References cited:
- EP-A1- 3 777 911
- WO-A1-2018/229504
- WO-A1-2021/084283
- US-A1- 2020 139 026
- US-B2- 8 702 646

## Description

The present invention relates to a breast pump shaped at least in part to fit inside a bra. The breast pump comprises a housing and a breast shield. The breast shield includes a nipple tunnel adapted to receive a nipple. The pump furthermore has a milk compartment adapted to contain a predetermined amount of expressed milk.

Such breast pump is e.g. known from WO 2018/229504 A1, which prior art breast pump includes six individual components which are to be assembled by the user prior to using the breast pump. Among those components, a housing element, a breast shield element, and a milk container element are the major components of the prior art breast pump. The breast shield element is detachable from the housing. The breast shield element is of transparent material and provides a cup-shaped pumping chamber housing adapted to be connected at its outer ring with a membrane element, which membrane element will be sandwiched between the breast shield element and the housing after inserting the breast shield element into the housing. The milk container element has an opening which is releasably closed by a separate cap element, which cap element is projected by a tube adapted to receive a valve element made of a soft elastomeric material adapted to sealingly cooperate with a nipple tunnel formed by the breast shield element to surround an outlet port formed at a circumference of the nipple tunnel.

A further breast pump according to the preamble of claim 1 is known from US 8,702,646 B2, which breast pump comprises an outer shell element and a breast shield element with a nipple tunnel. The prior art breast pump furthermore has a media separation element, which is attached to an inner closed end of the nipple tunnel, thereby sealingly surrounding an outlet port provided at a circumference of the nipple tunnel. A lower end of the media separation element is connected to a valve element leading to a milk compartment surrounded by the outer shell element. The upper end of the media separation element is adapted to connect to a hose leading to a vacuum source, which hose has to be inserted into a insertion hole at an outer circumference of the outer shell element.

A further prior art breast pump complying with the preamble of claim 1 is known from EP 3 777 911 A1. As the solution known from WO 2018/229504 A1, this prior art breast pump has a suction source in form of an electrically driven pump. The prior art breast pump has an outer shell element adapted to be connected to a pump module and housing a media separation element adapted to be connected to a valve element at its lower end and to a membrane element at its upper end and adapted to be secured to the inner close end of a nipple tunnel provided by a breast shield element.

Breast pumps as described in prior art are to be used by a breast feeding mother in order to extract milk. After expression of milk and feeding of the baby with the expressed milk, the components of the breast pump are disassembled for cleaning and sterilization purposes. This procedure is repeated after each individual use of the breast pump.

Thus, reuse of the breast pump by the user requires assembly of all components, which is troublesome with increasing number of components. Yet, the number of individual components of the breast pump are to be selected in view of the functionality of each of the components. Sealing the nipple tunnel against the milk compartment usually requires a valve made of an elastomeric material. Moreover, the membrane separating a working fluid chamber from a pumping chamber is likewise flexible and, thus, made of an adequate material while the outer shell element and the breast shield element are usually made from a rigid material. To allow visual control of the nipple arrangement within the nipple tunnel, at least parts of the breast pump may be made of a transparent material as described in WO 2018/229504 A1.

The present invention aims to provide a breast pump allowing economic manufacturing and use.

As a solution to the above object, the present invention provides a breast pump as defined in claims 1 and 2.

This breast pump is shaped to at least in part fit inside a bra. Thus, the breast pump of the present invention is a hands-free breast pump which can be used by the user by placing the breast pump into the bra. The bra holds the breast pump in place.

As known from prior art, the breast pump comprises a housing and a breast shield, which breast shield includes a nipple tunnel adapted to receive a nipple. The breast shield furthermore provides a milk compartment adapted to contain a predetermined amount of milk. For this, the milk compartment is in fluid connection with the nipple tunnel. As in prior art, the breast shield is detachable to allow cleaning of the breast shield, e.g. in a dishwasher. The pump furthermore comprises a working fluid chamber assigned to a vacuum source and a pumping chamber in fluid communication with the nipple tunnel. The working fluid chamber and the pumping chamber are fluidically separated from each other by a membrane, which membrane is usually made of a soft elastomeric material.

The vacuum source may be an external vacuum source. For a connection with such external vacuum source, the breast pump may provide an interface adapted to sealingly receive a tube. Alternatively, the vacuum source may be an electrically driven pump element, which pump element forms part of the breast pump. In such a case, the breast pump usually also comprises a source for electric energy in form of an electric accumulator which typically is rechargeable via an electric interface provided on the outer surface of the housing of the breast pump.

According to the present invention, the housing, the milk compartment, the working fluid chamber, and the pumping chamber are defined by only three components. Those components are unitary single-piece elements. Each of the components is a unitary element which does not require disassembly for cleaning purposes by the user. In fact, each of the three components is a unitary single-piece part which cannot be taken further part without destruction.

A breast pump of the present invention may comprise one or more seals, which seals typically are part of the respective unitary single-piece part. In the event such seal is a separate component, such seal will only seal the interface between at least two of the three components and will not per se define the milk compartment, the housing, the working fluid chamber or the pumping chamber. The seal will just prevent leakage of fluid from the respective compartment or chamber.

In the breast pump of the present invention, the working fluid chamber and the pumping fluid chamber are fluidically separated from each other by a membrane. The membrane typically is releasably attached to a working fluid chamber housing or a pumping chamber housing. In the assembled, i.e. use condition of the breast pump, the membrane is usually sandwiched between the working fluid chamber housing and the pumping chamber housing. Attachment of the membrane against one of the working fluid chamber housing or the pumping chamber housing is attained usually by a circumferential sealing rim of the membrane circumferentially surrounding an upper end or ring or rim of the pumping chamber housing or the working fluid chamber housing. In the use condition of the pump, an upper rim or ring of the other housing is laid against the membrane, thereby sandwiching and, thus, sealing the membrane between the working fluid chamber housing and the pumping chamber housing.

According to a parallel aspect of the present invention, there is provided a valve between the nipple tunnel and the milk compartment in order to avoid reflux of the milk within the milk compartment into the nipple tunnel in case the nipple tunnel due to the pumping activity of the breast pump has a lower pressure than the milk compartment. Such valve per se is known from the prior art solutions discussed above. According to the present invention, however, the membrane and the valve are formed by a media separation element. This media separation element is a unitary, i.e. single-piece part. Thus, the membrane and the valve can be manufactured as a single unitary part and handled for cleaning purposes as such unitary part. Manufacture and assembly/disassembly of the pump is greatly facilitated by having a single media separation element providing both functionalities, i.e. to separate the working fluid chamber from the pumping chamber and to avoid reflux from the milk compartment into the nipple tunnel.

The media separation element is preferably releasably arranged between an outer shell element and a breast shield element, which elements are unitary single-piece parts as described in connection with the first aspect of the present invention. The housing of the breast pump of the invention is typically formed by the breast shield element and the outer shell element, only.

Each of the elements per se can be manufactured of different materials. Sections of different materials may be glued or welded together. Welding is preferably attained by two-component injection molding, in which process a first section is molded in a first cavity, which first section is arranged or exposed in a second cavity, which second cavity is larger than the first cavity and filled with molten plastic material forming the second section, which second molten plastic material fuses with the first section, thereby forming a weld seam between the first and the second section in order to form a unitary element comprising the first and the second section.

The unitary element may have more than two sections. In order to allow visual control of nipple arrangement within the nipple tunnel, portions of in particular the outer shell element and/or the breast shield element may be transparent. Transparent in the meaning of the present invention shall be considered as a quality of material allowing a clear view through the material or a frosted transparent quality allowing visibility to a lesser extent but still sufficient to e.g. check proper position of the nipple within the nipple tunnel and/or milk flow or amount of expressed milk within the milk compartment. Other portions or sections of a single element may have a differing optical quality and may be opaque or frosted to a greater extent to essentially cover and thus optically shield functional parts within the breast pump. Such different optical qualities may be attained by use of different materials or working of a unitary element composed of a single material of one specific optical quality. Such working may include - without being limiting - painting or printing or layering with a decorative film or sleeve, edging or sandblasting of the surface of a specific component to alter the outer appearance of the component as manufactured by molding, in particular injection molding.

The components are usually made of plastic material, in particular thermoplastic material. The outer shell element and the breast shield element may be made of polypropylene or a thermoplastic polyester like Tritan^{™}, which material may be transparent in the meaning of the present invention or opaque.

According to preferred embodiment, the outer shell element has a first section covering the membrane and a second section lateral of the first section, which second section has a better transparent property than the first section and which second section allows the user to view the nipple tunnel through the outer shell element. In this preferred embodiment, the first and the second sections are preferably symmetrical relative to a vertical axis, which vertical axis extends perpendicular to a longitudinal axis of the nipple tunnel, which vertical axis lies within a plane intersecting a center of the membrane and/or a plane comprising the largest extension of the breast pump. In other words, the vertical axis may extend vertically in the field of gravity if the membrane and, thus, the pumping chamber and the working fluid chamber are arranged strictly above the nipple tunnel. In the use condition, the breast pump is laid against the left or the right breast in a slightly inclined orientation, in which the vertical axis is inclined outwardly such that the vertical axis is directed towards the bellybutton of the user and away from the head of the user. In such an inclined orientation, the user will view the nipple tunnel through a segment of the second section which is lateral of the first section and, thus, defines an outer circumferential portion of the outer shell element, while the first section is provided in the center and arranged symmetrically to the vertical axis and covers the membrane and, thus, the working fluid chamber and the pumping chamber, i.e. the technical parts of the breast pump used for the pumping operation.

The first section may be formed of a frosted transparent or opaque material which material has transparent properties not as good as the transparent properties of the second section, which second section may be slightly frosted or completely transparent to allow observation of milk flow or nipple orientation from the nipple tunnel into the milk compartment. As the second section is lateral to both sides of the first section, such observation can be carried out by the user no matter whether the breast pump is laid against the left or the right breast in the inclined orientation.

For the same reason, the nipple tunnel preferably is made of a material having better transparent materials than a flange section of the breast shield element, which flange section surrounds the nipple tunnel and at least in part defines a contact surface contacting the breast during use of the breast pump. Due to the improved transparent properties of the nipple tunnel, the user can view the nipple and, thus, milk flow within the nipple tunnel through the second section of the outer shell element. Moreover, in a disassembled stage, the user can confirm proper nipple alignment and, thus, selection of the appropriate size of the breast shield element conforming the individual anatomic constitution of the breast of the user. In practice, the breast pump may be manufactured and, thus, distributed with different sizes of breast shield elements making up a complete breast pump with a standardized outer shell element and a standardized media separation element. The individual size of the breast shield element provides standardized interfaces for sealingly connecting to the outer shell element and/or the membrane, i.e. media separation element.

According to a preferred embodiment of the present invention, which per se may define an inventive aspect and, thus, may define an inventive breast pump without the limitations of the other aspects, the walls defining the milk compartment are exclusively formed by the outer shell element and the breast shield element. Such a breast pump is usually shaped at least in part to fit inside a bra and comprises a housing, a breast shield including a nipple tunnel adapted to receive a nipple. The breast pump comprises a milk compartment adapted to contain a predetermined amount of expressed milk, wherein the breast shield is detachable from the housing. The pump will comprise a working fluid chamber assigned to a vacuum source and a pumping chamber in fluid communication with the nipple tunnel, wherein the working fluid chamber and the pumping chamber are fluidically separated from each other by a membrane. The pumping chamber and the working fluid chamber may be surrounded by the milk compartment. In such a case the pumping chamber and the working fluid chamber are usually formed by the outer shell element and the breast shield element, only. In such an embodiment, the membrane will be sandwiched between the outer shell element and the breast shield element and may at most form a seal between those elements but will not define the milk compartment. The same is true for any valve arranged between the nipple tunnel and the milk compartment. This valve will not define the milk compartment as such. It will only provide a seal between the milk compartment and the nipple tunnel.

In order to facilitate assembly of the breast pump of the present invention after cleaning and sterilization, the media separation element defining the membrane and the valve is secured to an inner end of the nipple tunnel. This inner end is usually a closed end. The inner end of the nipple tunnel has a securing interface adapted to hold the media separation element. For this, the media separation element usually has a securing opening adapted to be drawn over the inner end of the nipple tunnel for securing the media separation element against the breast shield element. After securing the media separation element in this way against the breast shield element, the membrane needs to be tilted only to properly align the membrane with an upper rim of a pumping chamber housing provided by the breast shield element to close the pumping chamber with the membrane. In the same way, the valve needs to be tilted, only to properly align the valve with a milk outlet having a port provided at an outer circumference of the nipple tunnel and usually opposite to the pumping chamber with respect to the longitudinal axis of the nipple tunnel.

According to a preferred embodiment of the present invention, the outer shell element defines a plug receptacle in communication with the working fluid chamber defined by the outer shell element between a circumferential rim provided of the outer shell element and the membrane. The plug receptacle is exposed at an outer periphery of the breast pump and adapted to receive a plug element. The plug element is releasably connected and sealed within the plug receptacle and provided with an interface for a suction source like a pump or a tube leading to a pump. On a regular basis, the interface is provided at a circumference of the plug element. The plug receptacle will allow arrangement of the plug element such that the interface either projects to one or another side relative to the vertical axis. Thus, proper alignment of the plug element will cope with the user's need for individually guiding the tube to one lateral side of the breast pump.

The plug element may be formed of silicone, PET or ABS. A front surface of the plug element is usually in line with the outer surface provided by the outer shell element. The plug receptacle preferably has a longish extension perpendicular to the vertical axis of the pump to allow lateral connection of the tube.

According to a preferred embodiment of the present invention, a snapping connection is provided between the breast shield element and the outer shell element, which snapping connection comprises mating snapping elements one of which being formed as a unitary segment of the outer shell element and the other being formed as a unitary segment of the breast shield element. The snapping connection allows assembly of the breast pump manually by the user and without any tooling. The snapping connection will secure the housing as an assembly formed by the breast shield element and the outer shell element.

Preferably, a sealing arrangement is provided circumferentially sealing an interface between the outer shell element and the breast shield element. Such sealing arrangement may comprise a seal made of a soft elastomeric material injection molded around the outer shell element or the breast shield element to securely seal the milk compartment and avoid spill of milk.

To allow easy disassembly of the breast pump, the breast shield element preferably has a wing defining a detachment surface allowing the user to remove the breast shield element from the outer shell element. Such wing is usually provided at a lower end of the breast pump, i.e. an end of the outer shell element opposite to the pumping chamber and the wording fluid chamber. To facilitate disassembly, the outer shell element at its outer surface will provide a recess allowing a thumb of the user to grasp behind the wing while another thumb may abut against the recess to hold the outer shell element while removing the breast shield element.

Further details and advantages of the present invention will become apparent from the following description of an embodiment in connection with the drawing. In the drawings:
- Fig. 1: is a perspective exploded view of the embodiment;
- Fig. 2: is a cross-sectional view of the embodiment of Fig. 1, which cross-sectional view contains the longitudinal axis of the nipple tunnel and extends parallel to a plane comprising the vertical axis;
- Fig. 3: is a perspective view of the front side of the embodiment of Figs. 1 and 2 and
- Fig. 4: is a front view showing two different use situations of the embodiment of Figs. 1 and 2.

In the Figures, reference numeral 100 identifies an outer shell element 100, reference numeral 200 identifies a media separation element and reference numeral 300 identifies a breast shield element.

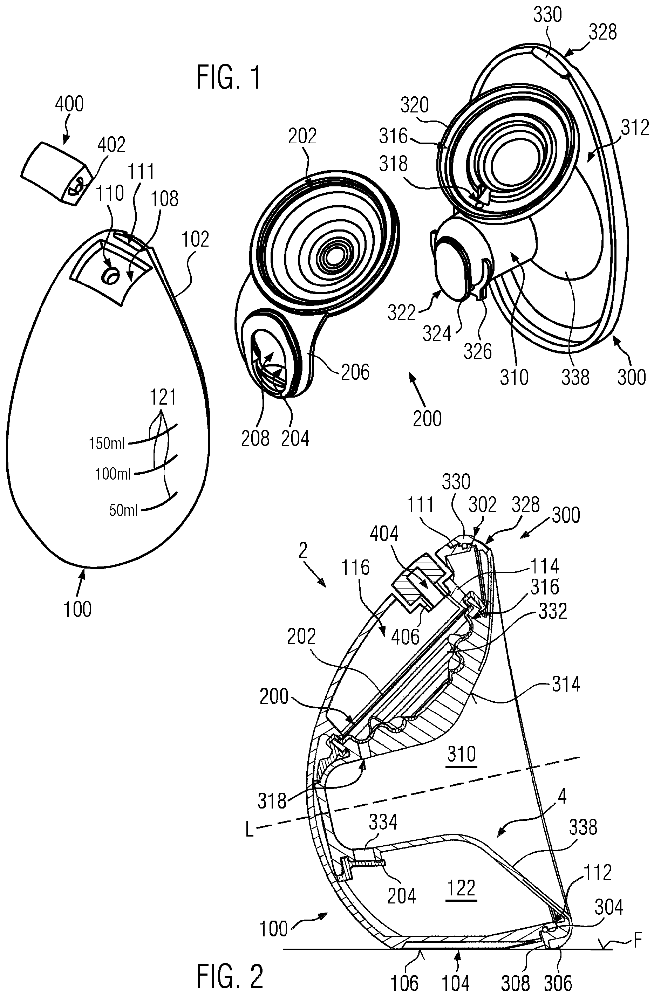

The outer shell element 100 is spherical and defines a plane interface 102 surrounded by a circumferential rim 302 of the breast shield element 300. A sealing arrangement in form of a circumferential seal 304 is provided between the interface 102 and the circumferential rim 302. At a bottom of the breast pump, the breast shield element 300 has a wing 306 defining a detachment surface 308. Adjacent to said detachment surface 308, the outer shell element 100 provides a flat recess 104 as an interruption to the spherical outer surface of the outer shell element 100, which recess 104 provides a standing surface 106. For opening the embodiment as depicted in Fig. 2, the user will grasp around the outer shell element 100 such that the thumb of the hand grasping around the outer shell element 100 will lay against the holding surface 106. The thumb of the other hand will cooperate with the detachment surface 308 while the fingers of the hand will at least in part cooperate with the circumferential rim 302 at the top of the pump to remove the breast shield element 300 from the outer shell element 100.

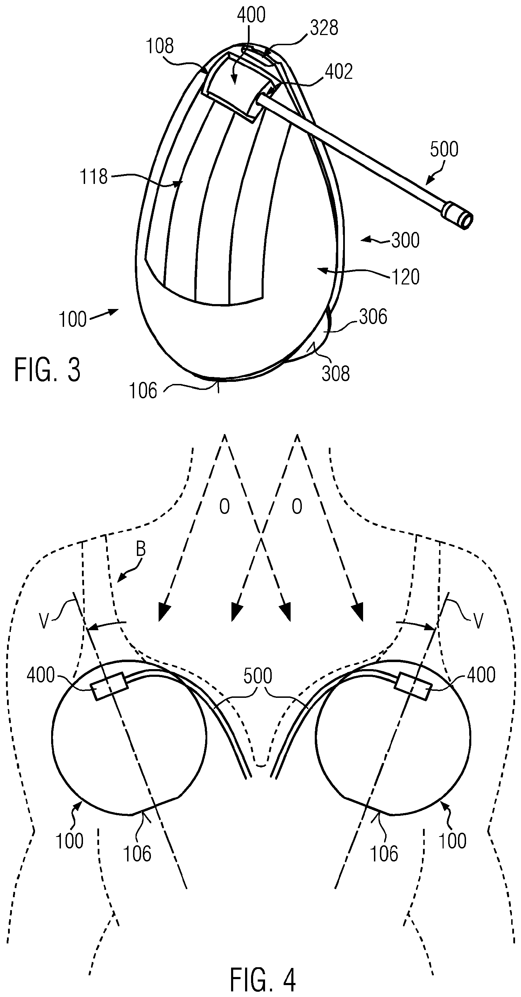

Near the top, the outer shell element 100 provides a plug receptacle 108 formed as a recess within the spherical outer surface and adapted to receive a plug element 400. The plug element 400 i.e. a circumference thereof is provided with a tube interface 402, which can be connected with a tube depicted in Fig. 3 and identified with reference numeral 500. The plug receptacle 108 has a longish extension. Due to the spherical constitution of the outer shell element 100, the opposing lateral rims surrounding the receptacle 108 are arranged lower than the axial extension of the tube interface 401. Thus, the tube 500 can be connected to the plug element 400 in a lateral extension as shown in Fig. 3.

The plug element 400 has a central bore 404 extended by a protrusion 406 sealingly cooperating with a working medium inlet 110 provided in the center of the plug receptacle 108 (compare Fig. 2). Thus, the plug element 400 can be arranged in an alignment 180° tilted around the working medium inlet to provide connection of the tube 500 in a lateral extension opposite to the extension shown in Fig. 3.

The breast shield element 300 of the present embodiment is a co-injection molded unitary element having a nipple tunnel 310 made of a transparent material and a flange section 312 made of an opaque material, which flange section 312 provides a contact surface 314 to at least in part contact the breast of the user. Due to the transparent properties of the nipple tunnel 310, the user can confirm proper alignment of the nipple within the nipple tunnel 310. Above the nipple tunnel 310, the breast shield element 300 defines a pumping chamber housing 316 which via a pumping inlet 318 is in fluid communication with the nipple tunnel 310. The pumping chamber 316 has a flat upper rim 320. The upper rim 320 is ring-shaped.

An inner end 322 of the nipple tunnel 310 is provided with a securing interface 324. The bottom side of the nipple tunnel 310 is projected by a valve receptacle 326.

At the top of the breast shield element 300, there is provided an open spout 328, which is projected by a snapping hook 330 provided by the breast shield element 300 and cooperating with a snapping counter surface 111 provided by a ring segment 112 of the outer shell element 100 with the circumferential seal interdisposed.

The media separation element 200 has a membrane 202 and a valve 204 being connected by a connection section 206 having a securing opening 208. The membrane 202, the valve 204 and the connection section 206 are realized in a single unitary element i.e. the media separation element 200.

As visible from Fig. 2, the outer shell element 100 has a circumferential rim 114 projecting inwardly from the concave inner surface of the outer shell element 100.

For assembly, the media separation element 200 will be secured to the inner end 322 of the nipple tunnel 310 by inserting the securing interface 324 at least in part into the securing opening 208. Mating connection surfaces of the media separation element 200 and the securing interface 324 will provide attachment of the two elements 100, 200. Thereafter, the membrane 202 will be tilted and secured against the upper rim 320 to close a pumping chamber 332. As a result and as visible from Fig. 2, the membrane 202 grasps around the upper rim 320 for proper attachment of the membrane 202 against the breast shield element 300, specifically the pumping chamber housing 316. On the other side, the valve 204 will be tilted to be connected within the valve receptacle 326 and abut against a milk outlet 334 provided on an outer circumference of the nipple tunnel 310.

In the further course of the assembly, the preassembled group comprising the media separation element 200 and the breast shield element 300 is inserted into the outer shell element 100. In the course of this assembly, the circumferential rim 114 will abut the membrane 202 so that the membrane 202 is sealed between the breast shield element 300 and the outer shell element 100.

Thus, a working fluid chamber 116 is provided within the outer shell element 100, which working fluid chamber 116 is in communication with the tube interface 402 via the working medium inlet 110.

Fig. 3 elucidates formation of the outer shell element 100 of two plastic materials with different transparent properties. For the following discussion, referral is made to a longitudinal axis of the nipple tunnel identified with reference numeral L in Figs. 2 and 3 and a vertical axis V extending perpendicular to the longitudinal axis L and extending in the same plane, which vertical axis defines the sectional view of Fig. 2. The embodiment is symmetrical with respect to the vertical axis V.

The same applies to the different sections of the outer shell element 100. A first section 118 covers the nipple tunnel 310 and, thus, is arranged in a central portion of the outer shell element 100. The first section 118 is made of a frosted transparent thermoplastic material. A second section 120 is provided laterally to the first section 118 and is made of slightly frosted transparent plastic material having a higher degree of transparency than the first section 118. The two sections 118, 120 are joined by co-injection molding. The lateral sides of the second section 120 are each provided with scale-lines 121 identifying the amount of milk collected within the pump. Alternatively scale-lines may be provided on the breast shield 4. In the present embodiment, the second section 120 is U-shaped and also extends over a central area of the outer shell element 100. In the present embodiment, the second section 120 provides the bottom of the outer shell element 100.

Fig. 1 exemplifies the breast shield element 300 being made of two different materials. The nipple tunnel 310 as well as a conical section 338 emerging from the nipple tunnel 310 are made of a clear transparent material, while the flange section 312 is made of a frosted or opaque plastic material. This constitution allows the user to check nipple alignment within the nipple tunnel 310 through the breast shield element 300.

In use, which is shown in Fig. 4, the breast pump is arranged with the vertical axis V slightly inclined outwardly below the breast pump. Thus, a view O of the user will be directed to the lateral sides of the outer shell element and, thus, the second section which will allow the user to observe milk flow and nipple alignment as well as e.g. valve operation. Fig. 4 also shows that the tube 500 for left and right breast use of the pump is directed towards the center of the body to be connected to an external pump thanks to the two different orientations in which the plug element 400 are rotated by 180° within the plug receptacle 108.

The outer shell 100 is avocado-shaped. The outer shell element 100 has the largest width approximately at level of the longitudinal axis L, the width being an extension perpendicular to the plane containing the vertical axis and the longitudinal axis. The outer shell element 100 and thus the breast shield element have the largest extension in height direction in the plane containing the vertical axis V. The longitudinal axis L is provided below the middle height of the outer shell element 100, the height being the extension parallel to the vertical axis. Due to this, the center of gravity of the pump is essentially leveled with the longitudinal axis or slightly below the longitudinal axis L. Due to this center of gravity, the pump is inclined to assume a positon in which the open spout 328 assumes the highest point of the pump, whereas the holding surface 106 assumes the lowest point. In fact and as evident from Fig. 2, this standing surface 106 securely supports an upright position of a pump being placed on a flat horizontal surface F.

Due to the avocado shape of the outer shell element 100, the breast pump of the embodiment can be stored at least in part within a bra B - Figure 4. Thus, the breast pump is a hands-free pump. In operation, the negative pressure of an external vacuum source which is not shown in the Figures cyclically evacuates the working fluid chamber 116, which causes the membrane 202 to expand either towards the working fluid chamber 116 or towards the pumping chamber 332. Through the pumping inlet, the pressure profile thus generated in the pumping chamber 332 is transferred into the nipple tunnel 310.

Due to this, milk is expressed, which milk is lead down into a milk compartment 122 sandwiched between the outer shell element 100 and the breast shield element 300. The milk compartment 122 in fact assumes the entire inner space within the outer shell element 100 which is not covered by material of the breast shield element 300 or the media separation element 200 or the volume of the working fluid chamber 116 and the pumping chamber 332. The milk compartment 122 is in communication with the atmosphere through the open spout 328. As the open spout 328 is arranged at the top of the breast pump and as the open spout 328 has a rather small dimension, spillage of milk expressed and contained within the milk compartment 122 can be avoided.

After expressing milk, the pump will be taken out of the bra B and can rest on the standing surface 106 in an upright position. The expressed milk can be poured out through the open spout 328 by rotating the pump upside down. During pumping operation and filling up the milk compartment 122 with milk, air can vent through the open spout 328.

In the present embodiment, a housing 2 of the breast pump is provided essentially by the outer shell element 100 and the breast shield element 300 only. The breast shield element 300 provides a breast shield 4 adapted to be laid against the beast of the user, specifically by means of the contact surface 314.

The outer shell element 100 and the breast shield element 300 are connected with each other. The outer periphery of breast shield element 300 is level with the outer periphery of the outer shell element 100. Free outer edges formed by the outer periphery of the breast shield element 300 and/or the outer shell element 100 are avoided in favor of a smooth outer housing 2 of the breast pump. The outer periphery of the outer shell element 100 is grasped around by and received within the outer periphery of breast shield element 300.

The working fluid chamber 116, the pumping chamber 332, and the milk compartment 122 are exclusively defined by the three individual but unitary elements discussed in detail above, i.e. the outer shell element 100, the media separation element 200, and the breast shield element 300. As the media separation element 200 as a single unitary element provides the membrane 202 as well as the valve 204, the breast pump of the present invention can be manufactured with a reduced number of parts. The media separation element 200 can easily be mounted on the breast shield element 300. Assembly of the pump is facilitated due to the reduced number of individual parts to be handled as well as the possibility to temporarily attach the media separation element 200 against the inner end 322 of the nipple tunnel 310. Due to the cooperating surfaces provided by the circumferential ring 114 and the upper rim 320, the membrane 202 is securely sandwiched to seal the fluid working chamber 116 and the pumping chamber 332 after assembly of the pump.

Transparent or slightly frosted sections of the pump allow visibility through the outer shell element 100 and at least portions of the breast shield element 300, in particular the nipple tunnel 310 to observe nipple alignment and/or milk flow and seize thereof indication termination of milk.

### List of Reference Signs

- 2: housing
- 4: breast shield
- 100: outer shell element
- 102: interface
- 104: recess
- 106: standing surface
- 108: plug receptacle
- 110: working medium inlet
- 111: snapping counter surface
- 112: ring segment
- 114: circumferential ring
- 116: working fluid chamber
- 118: first section
- 120: second section
- 121: scale line
- 122: milk compartment
- 200: media separation element
- 202: membrane
- 204: valve
- 206: connection section
- 208: securing opening
- 300: breast shield element
- 302: circumferential rim
- 304: circumferential seal
- 306: wing
- 308: detachment surface
- 310: nipple tunnel
- 312: flange section
- 314: contact surface
- 316: pumping chamber housing
- 318: pumping inlet
- 320: upper rim
- 322: inner end
- 324: securing interface
- 326: valve receptacle
- 328: open spout
- 330: snapping hook
- 332: pumping chamber
- 334: milk outlet
- 338: conical section
- 400: plug element
- 402: tube interface
- 404: bore
- 406: protrusion
- 500: tube
- L: longitudinal axis of the nipple tunnel
- V: vertical axis
- F: flat surface
- B: bra
- O: view

## Claims

1. A breast pump shaped at least in part to fit inside a bra (B) and comprising a housing (2), containing an outer shell element and a breast shield element, a breast shield (4) including a nipple tunnel (310) adapted to receive a nipple, a media separation element (200) and further comprising a milk compartment (122) adapted to contain a predetermined amount of expressed milk, further comprising a working fluid chamber (116) assigned to a vacuum source and a pumping chamber (332) in fluid communication with the nipple tunnel (310), wherein the working fluid chamber (116) and the pumping chamber (332) are fluidly separated from each other by a media separation element (200), **characterized in that**
the milk compartment (122), the working fluid chamber (116) and the pumping chamber (332) are constructed by the only three components, i.e. the outer shell element (100), the breast shield element (300) and the media separation element (200).

2. A breast pump shaped at least in part to fit inside a bra (B) and comprising a housing (2), a breast shield (4) including a nipple tunnel (310) adapted to receive a nipple and further comprising a milk compartment (122) adapted to contain a predetermined amount of expressed milk, further comprising a working fluid chamber (116) assigned to a vacuum source and a pumping chamber (332) in fluid communication with the nipple tunnel (310), wherein the working fluid chamber (116) and the pumping chamber (332) are fluidly separated from each other by a membrane (202), wherein a valve (204) is provided between the nipple tunnel (310) and the milk compartment (122) **characterized in that** the membrane (202) and the valve (204) are formed by a media separation element (200).

3. The breast pump according to claims 1 or 2, wherein the media separation element (200) is releasably arranged between an outer shell element (100) and a breast shield element (300).

4. The breast pump according to claim 2 or 3, wherein the media separation element (220) comprises a membrane (202) which is releasably sandwiched between an outer shell element (100) and a breast shield element (300) on one side of the nipple tunnel (310) and the valve (204), which is releasably attached to the breast shield element (300) on the other side of the nipple tunnel (310).

5. The breast pump according to any of the preceding claims, wherein the housing (2) is defined only by an outer shell element (100) and a breast shield element (300).

6. The breast pump according to any of the preceding claims, wherein the outer shell element (100) has a first section (118) covering the membrane (202) and a second section (120) lateral of the first section (118), which second section (120) has better transparent properties than the first section (118) allowing the user to view the nipple tunnel (310) through the outer shell element (100).

7. The breast pump according to any of the preceding claims, wherein the nipple tunnel (310) is made of a material having better transparent properties than a flange section (312) of the breast shield element (300), which flange section (312) surrounds the nipple tunnel (310) and at least in part defines a contact surface (314) for contacting the breast.

8. The breast pump according to any of the preceding claims, wherein the walls defining the milk compartment (122) are exclusively formed by an outer shell element (100) and a breast shield element (300).

9. The breast pump according to claim 1 further comprising a valve (204) defined by one of the three components.

10. The breast pump according to any of the claims 2 to 9, wherein the outer shell element (100), a media separation element (200) and a breast shield element (300) in combination define a working fluid chamber (116) assigned to a vacuum source, a pumping chamber (332) in fluid communication with the nipple tunnel (310) and the milk compartment (122).

11. The breast pump according to any of the preceding claims, wherein the media separation element (200) defining the membrane (202) and a valve (204) is secured to an inner end (322) of the nipple tunnel (310).

12. The breast pump according to any of the preceding claims, wherein the outer shell element (100) defines a plug receptacle (108) in communication with the working fluid chamber (116) and exposed on an outer periphery of the breast pump, which plug receptacle (108) is adapted to receive a plug element (400), which plug element (400) is releasably connected and sealed within the plug receptacle (108) and provided with an interface (402) for a suction source.

13. The breast pump according to any of the preceding claims further comprising a sealing arrangement (304) circumferentially sealing an interface between the outer shell element (100) and the breast shield element (300).

14. The breast pump according to any of the preceding claims further comprising a snapping connection comprising mating snapping elements (330; 111) one of which being formed as a unitary segment of the outer shell element (100) and the other being formed as a unitary segment of the breast shield element (300).

15. The breast pump according to any of the preceding claims, wherein the breast shield element (300) has a wing (306) defining a detachment surface (308) allowing the user to remove the breast shield element (300) from the outer shell element (100).

## Patentansprüche

1. Milchpumpe, die wenigstens teilweise so geformt ist, dass sie in einen Büstenhalter (B) passt, und umfassend ein Gehäuse (2), das ein äußeres Schalenelement sowie ein Brust-Abschirmungselement enthält, eine Brusthaube (4), die einen Brustwarzen-Tunnel (310) einschließt, der zum Aufnehmen einer Brustwarze eingerichtet ist, ein Medien-Trennelement (200) und des Weiteren umfassend einen Milchbehälter (122), der so eingerichtet ist, dass er eine vorgegebene Menge ausgepresster Milch aufnimmt, des Weiteren umfassend eine Arbeitsfluid-Kammer (116), die einer Vakuumquelle zugeordnet ist, sowie eine Pumpkammer (332), die in Fluidverbindung mit dem Brustwarzen-Tunnel (310) steht, wobei die Fluidverbindung zwischen der Arbeitsfluid-Kammer (116) und der Pumpkammer (332) mittels eines Medien-Trennelementes (200) getrennt ist,
**dadurch gekennzeichnet, dass**
der Milchbehälter (122), die Arbeitsfluid-Kammer (116) und die Pump-Kammer (332) durch die lediglich drei Komponenten, das heißt das äußere Schalenelement (100), das Brust-Abschirmungselement (300) und das Medien-Trennelement (200) gebildet werden.

2. Milchpumpe, die wenigstens teilweise so geformt ist, dass sie in einen Büstenhalter (B) passt, und umfassend ein Gehäuse (2), eine Brusthaube (4), die einen Brustwarzen-Tunnel (310) einschließt, der zum Aufnehmen einer Brustwarze eingerichtet ist, und des Weiteren umfassend einen Milchbehälter (122), der so eingerichtet ist, dass er eine vorgegebene Menge ausgepresster Milch aufnimmt, und des Weiteren umfassend eine Arbeitsfluid-Kammer (116), die einer Vakuumquelle zugeordnet ist, sowie eine Pumpkammer (332), die in Fluidverbindung mit dem Brustwarzen-Tunnel (310) steht, wobei die Fluidverbindung zwischen der Arbeitsfluid-Kammer (116) und der Pump-Kammer (332) mittels einer Membran (202) getrennt ist, ein Ventil (204) zwischen dem Brustwarzen-Tunnel (310) und dem Milchbehälter (122) vorhanden ist,
**dadurch gekennzeichnet, dass**
die Membran (202) und das Ventil (204) durch ein Medien-Trennelement (200) gebildet werden.

3. Milchpumpe nach Anspruch 1 oder 2, wobei das Medien-Trennelement (200) lösbar zwischen einem äußeren Schalenelement (100) und einem Brust-Abschirmungselement (300) angeordnet ist.

4. Milchpumpe nach Anspruch 2 oder 3, wobei das Medien-Trennelement (220) eine Membran (202), die lösbar zwischen einem äußeren Schalenelement (100) und einem Brust-Abschirmungselement (300) an einer Seite des Brustwarzen-Tunnels (310) eingeschlossen ist, sowie das Ventil (204) umfasst, das lösbar an der anderen Seite des Brustwarzen-Tunnels (310) an dem Brust-Abschirmungselement (300) angebracht ist.

5. Milchpumpe nach einem der vorangehenden Ansprüche, wobei das Gehäuse (2) lediglich durch ein äußeres Schalenelement (100) und ein Brust-Abschirmungselement (300) gebildet wird.

6. Milchpumpe nach einem der vorangehenden Ansprüche, wobei das äußere Schalenelement (100) einen ersten Teilabschnitt (118), der die Membran (202) abdeckt, sowie einen zweiten Teilabschnitt (120) seitlich von dem ersten Teilabschnitt (118) aufweist, und der zweite Teilabschnitt (120) bessere Transparenzeigenschaften aufweist als der erste Teilabschnitt (118), die es dem Benutzer ermöglichen, den Brustwarzen-Tunnel (310) durch das äußere Schalenelement (100) hindurch zu sehen.

7. Milchpumpe nach einem der vorangehenden Ansprüche, wobei der Brustwarzen-Tunnel (310) aus einem Material besteht, das bessere Transparenzeigenschaften aufweist als ein Flansch-Teilabschnitt (312) des Brust-Abschirmungselementes (300), wobei der Flansch-Teilabschnitt (312) den Brustwarzen-Tunnel (310) umschließt und wenigstens teilweise eine Kontaktfläche (314) zum Herstellen von Kontakt mit der Brust bildet.

8. Milchpumpe nach einem der vorangehenden Ansprüche, wobei die Wände, die den Milchbehälter (122) bilden, ausschließlich durch ein äußeres Schalenelement (100) und ein Brust-Abschirmungselement (300) gebildet werden.

9. Milchpumpe nach Anspruch 1, die des Weiteren ein Ventil (204) umfasst, das durch eine der drei Komponenten gebildet wird.

10. Milchpumpe nach einem der Ansprüche 2 bis 9, wobei das äußere Schalenelement (100), ein Medien-Trennelement (200) und ein Brust-Abschirmungselement (300) zusammen eine Arbeitsfluid-Kammer (116), die einer Vakuumquelle zugeordnet ist, eine Pump-Kammer (332), die in Fluidverbindung mit dem Brustwarzen-Tunnel (310) steht, und den Milchbehälter (122) bilden.

11. Milchpumpe nach einem der vorangehenden Ansprüche, wobei das Medien-Trennelement (200) die Membran (202) bildet und ein Ventil (204) an einem inneren Ende (322) des Brustwarzen-Tunnels (310) befestigt ist.

12. Milchpumpe nach einem der vorangehenden Ansprüche, wobei das äußere Schalenelement (100) eine Stopfen-Aufnahme (108) bildet, die in Verbindung mit der Arbeitsfluid-Kammer (116) steht und an einem Außenumfang der Milchpumpe freiliegt, wobei die Stopfen-Aufnahme (108) so eingerichtet ist, dass sie ein Stopfenelement (400) aufnimmt, wobei das Stopfenelement (400) lösbar mit der Stopfen-Aufnahme (108) verbunden und darin abgedichtet ist und mit einer Schnittstelle (402) für eine Unterdruckquelle versehen ist.

13. Milchpumpe nach einem der vorangehenden Ansprüche, die des Weiteren eine Dichtungsanordnung (304) umfasst, die eine Grenzfläche zwischen dem äußeren Schalenelement (100) und dem Brust-Abschirmungselement (300) am Umfang abdichtet.

14. Milchpumpe nach einem der vorangehenden Ansprüche, die des Weiteren eine Einrastverbindung umfasst, die ineinandergreifende Einrastelemente (330; 111) umfasst, von denen eines als ein Einzelsegment des äußeren Schalenelementes (100) ausgebildet ist und das andere als ein Einzelsegment des Brust-Abschirmungselementes (300) ausgebildet ist.

15. Milchpumpe nach einem der vorangehenden Ansprüche, wobei das Brust-Abschirmungselement (300) eine Lasche (306) aufweist, die eine Trennfläche (308) aufweist, die es dem Benutzer ermöglicht, das Brust-Abschirmungselement (300) von dem äußeren Schalenelement (100) zu entfernen.

## Revendications

1. Tire-lait façonné au moins en partie pour s'adapter à l'intérieur d'un soutien-gorge (B) et comprenant un boîtier (2), contenant un élément de coque externe et un élément de bouclier mammaire, un bouclier mammaire (4) incluant un tunnel de mamelon (310) adapté pour recevoir un mamelon, un élément de séparation des fluides (200) et comprenant en outre un compartiment à lait (122) adapté pour contenir une quantité prédéterminée de lait exprimé, comprenant en outre une chambre de fluide de travail (116) affectée à une source de vide et une chambre de pompage (332) en communication fluidique avec le tunnel de mamelon (310), dans lequel la chambre de fluide de travail (116) et la chambre de pompage (332) sont séparées fluidiquement l'une de l'autre par un élément de séparation des fluides (200),
**caractérisé en ce que**
le compartiment à lait (122), la chambre de fluide de travail (116) et la chambre de pompage (332) sont constitués des trois seuls composants, c'est-à-dire l'élément de coque externe (100), l'élément de bouclier mammaire (300) et l'élément de séparation des fluides (200).

2. Tire-lait façonné au moins en partie pour s'adapter à l'intérieur d'un soutien-gorge (B) et comprenant un boîtier (2), un bouclier mammaire (4) incluant un tunnel de mamelon (310) adapté pour recevoir un mamelon et comprenant en outre un compartiment à lait (122) adapté pour contenir une quantité prédéterminée de lait exprimé, comprenant en outre une chambre de fluide de travail (116) affectée à une source de vide et une chambre de pompage (332) en communication fluidique avec le tunnel de mamelon (310), dans lequel la chambre de fluide de travail (116) et la chambre de pompage (332) sont séparées fluidiquement l'une de l'autre par une membrane (202), dans lequel une vanne (204) est prévue entre le tunnel de mamelon (310) et le compartiment à lait (122) **caractérisé en ce que** la membrane (202) et la vanne (204) sont formées par un élément de séparation des fluides (200).

3. Tire-lait selon les revendications 1 ou 2, dans lequel l'élément de séparation des fluides (200) est agencé de manière amovible entre un élément de coque externe (100) et un élément de bouclier mammaire (300).

4. Tire-lait selon la revendication 2 ou 3, dans lequel l'élément de séparation des fluides (220) comprend une membrane (202) qui est prise en sandwich de manière libérable entre un élément de coque externe (100) et un élément de bouclier mammaire (300) d'un côté du tunnel de mamelon (310) et la vanne (204), qui est fixée de manière libérable à l'élément de bouclier mammaire (300) de l'autre côté du tunnel de mamelon (310).

5. Tire-lait selon l'une quelconque des revendications précédentes, dans lequel le boîtier (2) est défini uniquement par un élément de coque externe (100) et un élément de bouclier mammaire (300).

6. Tire-lait selon l'une quelconque des revendications précédentes, dans lequel l'élément de coque externe (100) présente une première section (118) recouvrant la membrane (202) et une deuxième section (120) latérale de la première section (118), cette deuxième section (120) présentant de meilleures propriétés de transparence que la première section (118) permettant à l'utilisateur de voir le tunnel de mamelon (310) à travers l'élément de coque externe (100).

7. Tire-lait selon l'une quelconque des revendications précédentes, dans lequel le tunnel de mamelon (310) est constitué d'un matériau ayant de meilleures propriétés de transparence qu'une section de bride (312) de l'élément de bouclier mammaire (300), cette section de bride (312) entoure le tunnel de mamelon (310) et définit au moins en partie une surface de contact (314) destinée à entrer en contact avec le sein.

8. Tire-lait selon l'une quelconque des revendications précédentes, dans lequel les parois définissant le compartiment à lait (122) sont exclusivement formées par un élément de coque externe (100) et un élément de bouclier mammaire (300).

9. Tire-lait selon la revendication 1 comprenant en outre une vanne (204) définie par l'un des trois éléments.

10. Tire-lait selon l'une quelconque des revendications 2 à 9, dans lequel l'élément de coque externe (100), un élément de séparation des fluides (200) et un élément de bouclier mammaire (300) définissent en combinaison une chambre de fluide de travail (116) affectée à une source de vide, une chambre de pompage (332) en communication fluidique avec le tunnel de mamelon (310) et le compartiment à lait (122).

11. Tire-lait selon l'une quelconque des revendications précédentes, dans lequel l'élément de séparation des fluides (200) définissant la membrane (202) et une vanne (204) est fixée à une extrémité intérieure (322) du tunnel de mamelon (310).

12. Tire-lait selon l'une quelconque des revendications précédentes, dans lequel l'élément de coque externe (100) définit un réceptacle de bouchon (108) en communication avec la chambre de fluide de travail (116) et exposé sur une périphérie externe du tire-lait, lequel réceptacle de bouchon (108) est adapté pour recevoir un élément de bouchon (400), lequel élément de bouchon (400) est connecté de manière libérable et scellé dans le réceptacle de bouchon (108) et prévu avec une interface (402) pour une source d'aspiration.

13. Tire-lait selon l'une quelconque des revendications précédentes comprenant en outre un agencement d'étanchéité (304) scellant de manière circonférentielle une interface entre l'élément de coque externe (100) et l'élément de bouclier mammaire (300).

14. Tire-lait selon l'une quelconque des revendications précédentes comprenant en outre une liaison par encliquetage comprenant des éléments d'encliquetage appariés (330 ; 111) dont l'un est formé d'un segment unitaire de l'élément de coque externe (100) et l'autre est formé d'un segment unitaire de l'élément de bouclier mammaire (300).

15. Tire-lait selon l'une quelconque des revendications précédentes, dans lequel l'élément de bouclier mammaire (300) a une aile (306) définissant une surface de détachement (308) permettant à l'utilisateur de retirer l'élément de bouclier mammaire (300) de l'élément de coque externe (100).
